(19)

Europäisches
Patentamt

European
Patent Office

Office européen
des brevets

(11)  **EP 3 741 294 B1**

(12)  **EUROPEAN PATENT SPECIFICATION**

(45) Date of publication and mention
of the grant of the patent:
**23.11.2022 Bulletin 2022/47**

(21) Application number: **18901747.8**

(22) Date of filing: **18.01.2018**

(51) International Patent Classification (IPC):
**A61B 5/316** $^{(2021.01)}$

(52) Cooperative Patent Classification (CPC):
**A61B 5/316; A61B 5/318; A61N 1/39**

(86) International application number:
**PCT/CN2018/073250**

(87) International publication number:
**WO 2019/140603 (25.07.2019 Gazette 2019/30)**

(54) **ECG SIGNAL DENOISING COMPUTER PROGRAM, STORAGE MEDIUM, AND TERMINAL**

COMPUTERPROGRAMM ZUR EKG-SIGNALRAUSCHUNTERDRÜCKUNG, SPEICHERMEDIUM
UND ENDGERÄT

PROGRAMME D'ORDINATEUR DE DÉBRUITAGE DE SIGNAL D'ECG, MÉDIUM DE STOCKAGE,
ET TERMINAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**25.11.2020 Bulletin 2020/48**

(73) Proprietors:
• **Shenzhen Mindray Bio-Medical Electronics Co.,
Ltd**
**Guangdong 518057 (CN)**
• **Shenzhen Mindray Scientific Co., Ltd.**
**Shenzhen, Guangdong 518000 (CN)**

(72) Inventors:
• **HONG, Junbiao**
**Shenzhen, Guangdong 518057 (CN)**
• **JIANG, Haoyu**
**Shenzhen, Guangdong 518057 (CN)**
• **HU, Mimi**
**Shenzhen, Guangdong 518057 (CN)**

• **YE, Wenyu**
**Shenzhen, Guangdong 518057 (CN)**

(74) Representative: **KIPA AB**
**P O Box 1065**
**251 10 Helsingborg (SE)**

(56) References cited:
**CN-A- 104 323 769      CN-A- 104 363 823
CN-A- 107 249 684      US-A1- 2014 100 466
US-A1- 2015 165 223    US-B2- 9 289 134**

• **IRUSTA U ET AL: "A Least Mean-Square Filter for
the Estimation of the Cardiopulmonary
Resuscitation Artifact Based on the Frequency of
the Compressions", IEEE TRANSACTIONS ON
BIOMEDICAL ENGINEERING, IEEE SERVICE
CENTER, PISCATAWAY, NJ, USA, vol. 56, no. 4,
1 April 2009 (2009-04-01), pages 1052-1062,
XP011342929, ISSN: 0018-9294, DOI:
10.1109/TBME.2008.2010329**

**Description**

Technical Field

The present application relates to the technical field of medical signal processing, and in particular to an ECG signal denoising method and a terminal.

Background Art

In clinical practice, when a doctor performs cardiopulmonary resuscitation (CPR) compression on a patient, great interference will be posed to an electrocardiograph (ECG) signal to seriously affect the quality of the ECG signal, such that the doctor can hardly acquire the patient's physical condition information easily from the ECG signal, and has to stop the CPR compression to acquire the patient's physical condition information from the ECG signal, which increases the interruption time of the CPR compression and reduces the success rate of rescue.

For various ECG signals, the existing technologies all adopt fixed-order adaptive filters, causing the following problems:

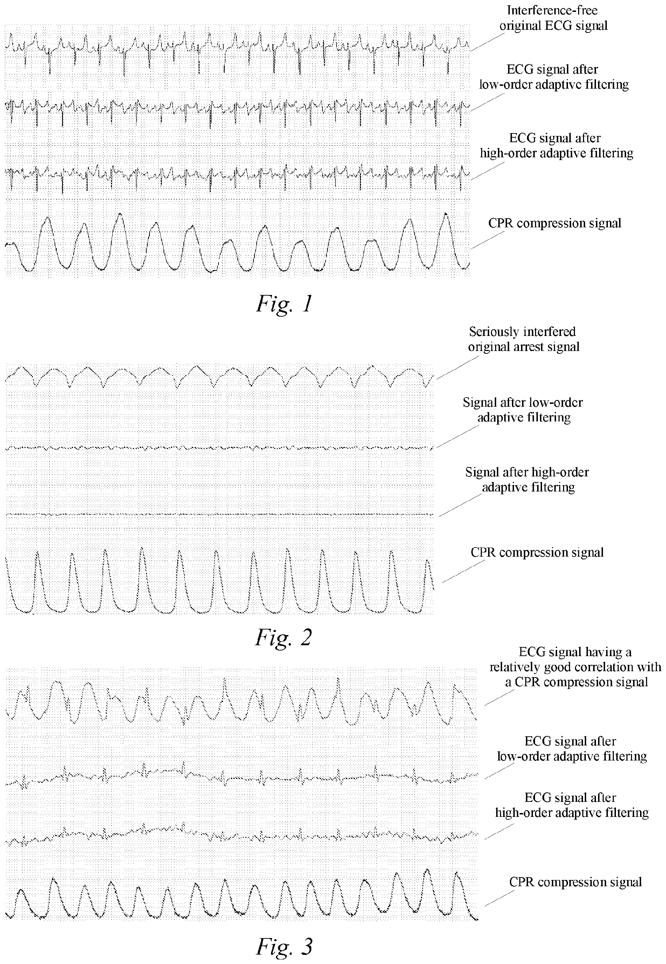

1. In clinical practice, CPR compression is performed sometimes, but it does not interfere with the ECG signal. In this case, if filtering is still performed, the ECG signal will be seriously distorted. As shown in Fig. 1, interference does not exist in the ECG signal originally, but the ECG signal is distorted relatively seriously after adaptive filtering.
2. As shown in Fig. 2, the ECG signal is an arrest signal. If a low-order adaptive filter is used, there will be significant residuals in the filtered ECG signal, which affects the doctor's judgment on the rhythm type, so only a high-order adaptive filter can completely eliminate interference and restore the arrest signal.
3. As shown in Fig. 3, the ECG signal is an autonomic rhythm signal. If a high-order adaptive filter is used, a signal in the form of tremor waves will be introduced. In this case, some automatic analysis algorithms for an arrhythmia may misjudge this signal as a ventricular fibrillation signal, so a low-order adaptive filter is used to effectively filter the interference introduced by CPR compression and also retain the characteristics of the ECG signal.

US9289134B2 (ZOLL MEDICAL CORP [US]) discloses that an appropriate analysis method can be selected according to the strength of the cross-correlation between the ECG signal and the CPR signal. A low cross-correlation value between the ECG signal and the CPR signal typically indicates that the ECG analysis performed during CPR may be less reliable. Due to the less reliability of the ECG analysis, the system can utilize a longer period of CPR-free time in a re-confirmation analysis.

Summary of the Invention

The present application provides an ECG signal denoising method and a terminal, which can achieve a better filtering effect.

A computer program in accordance with the invention is defined in claim 1. A terminal in accordance with the invention is defined in claim 9. Any method does not form part of the claimed invention.

The present application provides an ECG signal denoising method and a terminal. First, the terminal detects a CPR signal and a first signal, wherein the first signal comprises an ECG signal and noise of the ECG signal. Second, the terminal analyzes whether the noise of the ECG signal in the first signal correlates with the CPR signal. Then, if the noise of the ECG signal correlates with the CPR signal, the terminal determines, according to a degree of the correlation between the noise of the ECG signal and the CPR signal, a filtering method for the first signal. Finally, the terminal filters the first signal according to the determined filtering method to remove the noise of the ECG signal. By using the present application, a better filtering effect can be achieved.

Brief Description of the Drawings

In order to illustrate the technical solutions in the embodiments of the present application more clearly, a brief introduction to the drawings required for the embodiments will be provided below. Obviously, the drawings in the following description are merely some of the embodiments of the present application, and those of ordinary skill in the art would also have been able to obtain other drawings according to these drawings without involving any inventive effort.

Figs. 1 to 3 are schematic diagrams of ECG signal denoising provided by the present application;
Fig. 4 is a schematic flowchart of an ECG signal denoising method provided by the present application;
Fig. 5 is a schematic structural diagram of an adaptive filter provided by the present application;

Fig. 6 is a schematic structural diagram of a filter provided by the present application;

Fig. 7 is a schematic flowchart of another ECG signal denoising method provided by the present application;

Fig. 8 is a schematic flowchart of still another ECG signal denoising method provided by the present application;

Fig. 9 is a schematic flowchart of still another ECG signal denoising method provided by the present application;

Fig. 10 is a schematic structural diagram of a terminal provided by the present application; and

Fig. 11 is a schematic structural diagram of another terminal provided by the present application.

Detailed Description of Embodiments

The technical solutions of the present application will be described below clearly and comprehensively in conjunction with the drawings of the present application. Clearly, the embodiments described are some embodiments of the present application and are not all the possible embodiments. Based on the embodiments given in the present application, all other embodiments that would be obtained by those of ordinary skill in the art without expending inventive effort shall all fall within the scope of protection of the present application, where the scope is defined by the claims.

It should be understood that when used in the description and the appended claims, the terms "comprise" and "contain" indicate the presence of the described features, wholes, steps, operations, elements and/or the components, but do not exclude the presence or addition of one or more other features, wholes, steps, operations, elements, components and/or the combinations thereof.

It should also be understood that the terms used in the description of the present application are only intended to describe specific embodiments, but not to limit the present application. As used in the description and the appended claims of the present application, unless the context clearly indicates otherwise, the singular forms "a", "an" and "the" are intended to comprise the plural forms.

It should also be further understood that the term "and/or" used in the description and the appended claims of the present application refers to one of the items listed correlatively or any combination and all possible combinations of the items, and comprises these combinations.

As used in the description and the appended claims, the term "if' may be interpreted as "when" or "once" or "in response to determination" or "in response to detection" according to the context. Similarly, the phrase "if determined" or "if detected [described condition or event]" may be interpreted as "once determined" or "in response to determination" or "once detected [described condition or event]" or "in response to detection of [the described condition or event]" according to the context.

In specific implementations, the terminal described in the present application includes, but is not limited to, other portable apparatuses such as a mobile phone, a laptop computer, or a tablet computer with a touch-sensitive surface (e.g., a touch screen display and/or a touch pad). It should also be understood that, in some embodiments, the apparatus is not a portable communication apparatus, but a desktop computer with a touch-sensitive surface (e.g., a touch screen display and/or a touch pad).

A terminal comprising a display and a touch-sensitive surface is described in the following discussion. However, it should be understood that the terminal may comprise one or more other physical user interface apparatuses such as a physical keyboard, a mouse, and/or a joystick.

The terminal supports various application programs, such as one or more of the following: drawing application programs, presentation application programs, word processing application programs, website creation application programs, disk burning application programs, spreadsheet application programs, game application programs, phone application programs, video conferencing application programs, e-mail application programs, instant messaging application programs, exercise support application programs, photo management application programs, digital camera application programs, digital video camera application programs, web browsing application programs, digital music player application programs, and/or digital video player application programs.

Various application programs executable on the terminal may employ at least one common physical user interface apparatus such as a touch-sensitive surface. One or more functions of the touch-sensitive surface and corresponding information displayed on the terminal may be adjusted and/or changed between the application programs and/or within the corresponding application programs. In this way, the common physical architecture (e.g., the touch-sensitive surface) of the terminal may support various application programs with user interfaces that are intuitive and transparent to users.

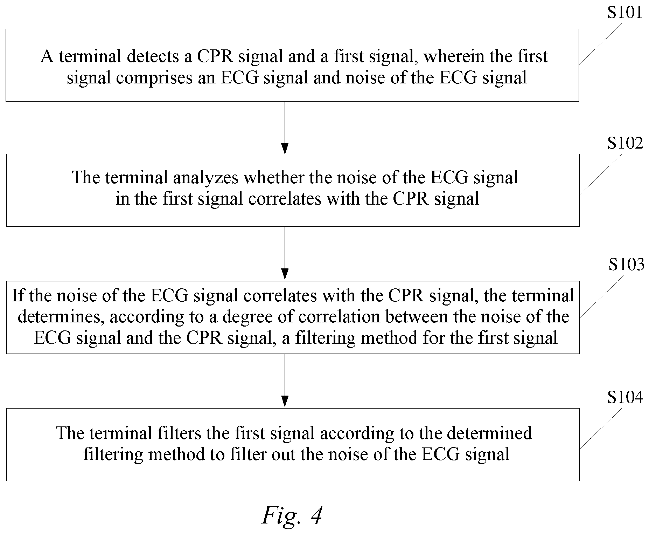

Fig. 4 is a schematic flowchart of an ECG signal denoising method provided by the present application. As shown in Fig. 1, the method may comprise at least the following steps:

S 101, a terminal may detect a CPR signal and a first signal. Here, the first signal may comprise an ECG signal and noise of the ECG signal.

In this embodiment of the present application, the CPR signal may include at least but is not limited to the following signals: a CPR compression pressure signal, a CPR compression displacement signal, a CPR compression acceleration signal or a chest impedance signal, etc. It should be noted that the frequency range of CPR compression may include but is not limited to 1 Hz to 5 Hz.

It may be known that the terminal may monitor or detect, by means of a CPR monitor, the CPR signal from a CPR sensor placed on a user's body. In addition, the terminal may monitor or detect, by means of the ECG monitor, the ECG signal from an ECG sensor placed on the user's body.

It may be understood that, in addition to the ECG signal, the terminal may monitor or detect, by means of the ECG sensor, the noise of the ECG signal from the ECG sensor placed on the user's body, and the noise may be generated by the CPR compression. In this embodiment of the present application, the signal from the ECG sensor placed on the user's body is collectively referred to as a first signal, and the first signal may include but is not limited to the ECG signal and the noise of the ECG signal.

It should be noted that the noise of the ECG signal may comprise at least the following two types of noise.

First type of noise: noise of the ECG signal caused by a CPR process.

Second type of noise: noise of the ECG signal caused by other factors in the environment (other than the CPR process).

S102, the terminal may analyze whether the noise of the ECG signal in the first signal correlates with the CPR signal.

In this embodiment of the present application, the determination of a first signal component of the CPR signal may comprise at least the following steps:

Step 1: the terminal may perform frequency domain analysis (power spectrum estimation) on the CPR signal.
Step 2: the terminal may determine a first frequency of the CPR signal from a power spectrum estimation diagram of the CPR signal according to a first preset value.

Optionally, the first frequency may be a center frequency of the CPR signal. Here, the center frequency of the CPR signal is a frequency at which a peak with the maximum amplitude in the power spectrum estimation diagram of the CPR signal is located. That is, the terminal may determine the center frequency of the CPR signal from the power spectrum estimation diagram of the CPR signal according to the first preset value.

Step 3: the terminal may determine a corresponding signal within a range of 0.5 Hz centered on the center frequency of the CPR signal as the first signal component of the CPR signal.

It should be noted that the frequency variation range of the first signal may be 0.5 Hz to 40 Hz. The frequency variation range of the CPR signal may be 0.5 Hz to 40 Hz.

Here, the determination of the noise of the ECG signal caused by the CPR process may comprise at least the following steps:

Step 1: the terminal may perform frequency domain analysis (power spectrum estimation) on the CPR signal.
Step 2: the terminal may determine a first frequency of the CPR signal from a power spectrum estimation diagram of the CPR signal according to a first preset value. Here, the first frequency may be a center frequency of the CPR signal.
Step 3: the terminal may determine a corresponding signal, in the first signal, within a range of 0.5 Hz centered on the center frequency of the CPR signal as the noise of the ECG signal caused by the CPR process.

It may be understood that the terminal may determine a second frequency of the first signal from a power spectrum estimation diagram of the first signal according to a second preset value. Here, the second frequency may be a center frequency of the first signal. It should be noted that the center frequency of the first signal is a frequency at which a peak with the maximum amplitude in the power spectrum estimation diagram of the first signal is located.

Specifically, the terminal may analyze, by at least the following three methods, whether the noise of the ECG signal correlates with the CPR signal.

First method: if the frequency interval between the center frequency of the first signal and the center frequency of the CPR signal is smaller than a first threshold, the terminal may determine that the noise of the ECG signal correlates with the CPR signal. Here, the first threshold is controllable, and the value range of the first threshold may be [1, 2].

Specifically, the frequency interval $f$ between the center frequency of the first signal and the center frequency of the CPR signal may be:

$$f = \|\text{the center frequency } f_1 \text{ of the first signal} - \text{the center frequency } f_2 \text{ of the CPR signal}\| \tag{1}$$

That is, if the frequency interval $f$ is smaller than the first threshold, the terminal may determine that the noise of the ECG signal correlates with the CPR signal.

Second method: if the ratio of the noise of the ECG signal caused by the CPR process to the first signal is greater than a second threshold, the terminal may determine that the noise of the ECG signal correlates with the CPR signal, wherein the value range of the second threshold may be [0.35, 0.4].

Specifically, the ratio $\theta_1$ of the noise of the ECG signal caused by the CPR process to the first signal may be:

$$\theta_1 = \text{the sum of amplitudes of a power spectrum curve of the first signal within}$$
$$\text{the range ([the center frequency } f_2 \text{ of the CPR signal - 0.5 Hz, the center frequency}$$
$$f_2 \text{ of the CPR signal + 0.5 Hz]) / (the sum of amplitudes of the power spectrum}$$
$$\text{curve of the first signal within the range [0.5 Hz, 40 Hz])} \tag{2}$$

That is, if the ratio $\theta_1$ is greater than the second threshold, the terminal may determine that the noise of the ECG signal correlates with the CPR signal.

Third method: if the ratio of the noise of the ECG signal caused by the CPR process to the first signal is greater than a third threshold and the frequency interval between the center frequency of the CPR signal and the center frequency of the first signal is smaller than a fourth threshold, the terminal may determine that the noise of the ECG signal correlates with the CPR signal. Here, the value range of the third threshold may be [0.25, 0.3], and the value range of the fourth threshold may be [1, 2].

That is, if the frequency interval $f$ is smaller than the fourth threshold and the ratio $\theta_1$ is greater than the third threshold, the terminal may determine that the noise of the ECG signal correlates with the CPR signal.

In summary, the terminal may ascertain whether the noise of the ECG signal correlates with the CPR signal, according to the frequency interval between the center frequency of the first signal and the center frequency of the CPR signal and/or the ratio of the noise of the ECG signal caused by the CPR process to the first signal.

S 103, if the noise of the ECG signal correlates with the CPR signal, the terminal determines according to a degree of the correlation between the noise of the ECG signal and the CPR signal, a filtering method for the first signal.

The method for filtering the first signal by the terminal includes the following two methods:

First filtering method: the terminal filters the first signal by means of a high-order adaptive filter to remove the noise of the ECG signal. Here, the order of the high-order adaptive filter may be 4 to 6.

Second filtering method: the terminal filters the first signal by means of a low-order adaptive filter to remove the noise of the ECG signal. Here, the order of the low-order adaptive filter may be 2 to 3.

Specifically, the terminal determines the filtering method for the first signal depending on whether the degree of the correlation between the noise of the ECG signal and the CPR signal exceeds a first value. If the terminal determines that the degree of the correlation between the noise of the ECG signal and the CPR signal exceeds the first value, the terminal determines to use the first filtering method for filtering the first signal. Otherwise, the terminal determines to use the second filtering method for filtering the first signal.

It should be noted that, compared with the second filtering method, the first filtering method may filter more noise of the ECG signal, but may also remove part of useful signal (ECG signal) in the first signal. The capability of the second filtering method (low-order adaptive filter) to remove the noise of ECG is lower than that of the first filtering method (high-order adaptive filter), but may retain more ECG signals (avoid filtering out too many ECG signals in the first signal).

Further, the method that the terminal ascertains whether the degree of the correlation between the noise of the ECG signal and the CPR signal exceeds the first value may comprise:

if the difference between the ratio of the noise of the ECG signal caused by the CPR process to the first signal and the ratio of the first signal component of the CPR signal is smaller than a fifth threshold, the terminal determines that the degree of the correlation between the noise of the ECG signal and the CPR signal exceeds the first value; or otherwise, the terminal determines that the degree of the correlation between the noise of the ECG signal and the CPR signal does not exceed the first value.

The difference between the ratio of the noise of the ECG signal caused by the CPR process to the first signal and the ratio of the first signal component of the CPR signal is specifically described below. Specifically, the ratio $\theta_2$ of the first signal component of the CPR signal may be:

$$\theta_2 = \text{the sum of amplitudes of a power spectrum curve of the CPR signal within}$$

the range ([the center frequency $f_2$ of the CPR signal - 0.5 Hz, the center frequency

$f_2$ of the CPR signal + 0.5 Hz]) / (the sum of amplitudes of the power spectrum curve

of the CPR signal within the range [0.5 Hz, 40 Hz]) (3)

In combination with the ratio $\theta_1$ of the noise of the ECG signal caused by the CPR process to the first signal in formula (2), the difference $\theta$ between the ratio $\theta_1$ of the noise of the ECG signal caused by the CPR process to the first signal and the ratio $\theta_2$ of the first signal component of the CPR signal may be:

$$\theta = \left\| \theta_1 - \theta_2 \right\|$$ (4)

That is, if the difference $\theta$ is smaller than the fifth threshold, the terminal determines that the degree of the correlation between the noise of the ECG signal and the CPR signal exceeds the first value. Otherwise, the terminal determines that the degree of the correlation between the noise of the ECG signal and the CPR signal does not exceed the first value.

It may be understood that the fifth threshold is controllable, and the terminal may determine the filtering method suitable for the first signal by setting the fifth threshold. It should be noted that the value range of the fifth threshold may be [0.25, 0.3].

S 104, the terminal may filter the first signal according to the determined filtering method to remove the noise of the ECG signal.

In this embodiment of the present application, the terminal may filter the first signal according to the determined filtering method (the first filtering method or the second filtering method), that is, the terminal may filter the first signal according to the determined adaptive filter (the high-order adaptive filter or the low-order adaptive filter).

It should be noted that parameters of the adaptive filter may be changed by using an adaptive algorithm, to remove the noise of the ECG signal.

Here, the adaptive algorithm may include but is not limited to the following three algorithms:
First algorithm: least mean square (LMS) algorithm.

Second algorithm: steepest descent method.

Third algorithm: recursive least squares (RLS) algorithm.

The adaptive filter may comprise at least the following two basic processes:
Filtering process: the terminal may filter, by means of the filter, the CPR signal that causes the noise of the ECG signal.

Adaptive process: the terminal automatically adjusts the parameters of the filter (e.g., a weight of the filter) by using the adaptive algorithm and the filtered CPR signal.

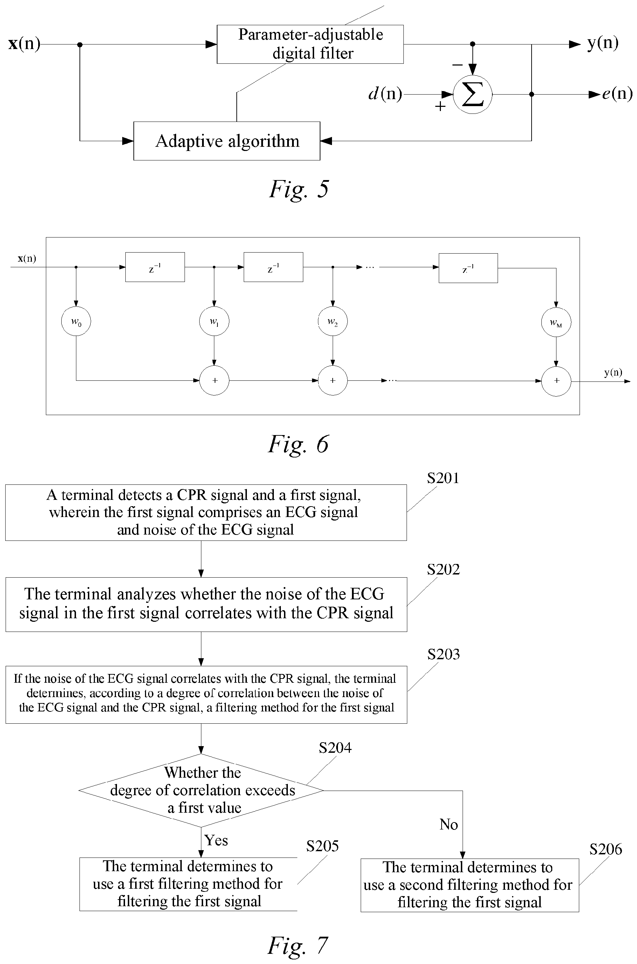

Fig. 5 exemplarily shows a schematic structural diagram of an adaptive filter. As shown in Fig. 5, x(n) represents the CPR signal (an input vector at the moment n, that is, a column vector composed of input data entering each tap of the adaptive filter at the moment n), $d$(n) represents the ECG signal, y(n) represents the filtered CPR signal, and $e$(n) represents the filtered ECG signal.

It may be understood that the filtered CPR signal may be expressed as:

$$y(n) = \mathbf{x}^{\mathrm{T}}(n)\mathbf{w}(n)$$ (5)

where $\mathbf{w}$(n) represents a tap weight vector at the moment n (that is, a column vector composed of each tap weight of the adaptive filter at the moment n).

The filtered ECG signal may be expressed as:

$$e(\mathrm{n}) = d(\mathrm{n}) - y(\mathrm{n})$$ (6)

The tap weight vector **w**(n) of the adaptive filter may be expressed as:

$$\mathbf{w}(n+1) = \mathbf{w}(n) + 2\mu e(n)\mathbf{x}(n) \tag{7}$$

Here, the value range of the step factor $\mu$ may be (0.0000002, 0.00002).

Further, the parameter-adjustable digital filter as shown in Fig. 5 may be a transversal filter as shown in Fig. 6 (a tapped delay line filter or a finite impulse response filter).

As shown in Fig. 6, it should be known that $z^{-1}$ represents a unit delay unit, and the number of unit delay units may be the order of the adaptive filter. n represents the moment, and M represents the order of the adaptive filter. $w_m^*$ represents a parameter of the adaptive filter or the weight of the adaptive filter.

It may be understood that formula (5) that characterizes the filtered CPR signal may also be expressed as:

$$y(n) = \sum_{m=0}^{M} w_m^* x(n-m) \tag{8}$$

Here, $x(n - m)$ represents the CPR signal on the $m^{th}$ tap line of the adaptive filter at the moment n.

In summary, after the terminal sets the order of the adaptive filter (e.g., the number of delay units in the adaptive filter), the terminal may automatically adjust the parameters of the adaptive filter (i.e., the weight of the adaptive filter) by using the adaptive algorithm and the filtered CPR signal to minimize the CPR signal, that is, the terminal may remove the noise (noise caused by the CPR process) of the ECG signal in the first signal to the greatest extent by means of the adaptive filter.

Figs. 5 and 6 are only used to explain the present application, and should not constitute limitations.

Fig. 7 is a schematic flowchart of another ECG signal denoising method provided by the present application. The method embodiment of Fig. 4 is specifically described. As shown in Fig. 7, the method may comprise at least the following steps:

S201, a terminal detects a CPR signal and a first signal, wherein the first signal may comprise an ECG signal and noise of the ECG signal.

In this embodiment of the present application, the noise of the ECG signal may comprise at least the following two types of noise.

First type of noise: noise of the ECG signal caused by a CPR process.

Second type of noise: noise of the ECG signal caused by other factors in the environment (other than the CPR process).

S202, the terminal analyzes whether the noise of the ECG signal in the first signal correlates with the CPR signal.

In this embodiment of the present application, the determination of a first signal component of the CPR signal may comprise at least the following steps:

Step 1: the terminal may perform frequency domain analysis (power spectrum estimation) on the CPR signal.

Step 2: the terminal may determine a first frequency of the CPR signal from a power spectrum estimation diagram of the CPR signal according to a first preset value. Here, the first frequency may be a center frequency of the CPR signal. Here, the center frequency of the CPR signal is a frequency at which a peak with the maximum amplitude in the power spectrum estimation diagram of the CPR signal is located. That is, the terminal may determine the center frequency of the CPR signal from the power spectrum estimation diagram of the CPR signal according to the first preset value.

Step 3: the terminal may determine a corresponding signal within a range of 0.5 Hz centered on the center frequency of the CPR signal as the first signal component of the CPR signal.

It should be noted that the frequency variation range of the first signal may be 0.5 Hz to 40 Hz. The frequency variation range of the CPR signal may be 0.5 Hz to 40 Hz.

Here, the determination of the noise of the ECG signal caused by the CPR process may comprise at least the following steps:

Step 1: the terminal may perform frequency domain analysis (power spectrum estimation) on the CPR signal.

Step 2: the terminal may determine a first frequency of the CPR signal from a power spectrum estimation diagram of the CPR signal according to a first preset value. Here, the first frequency may be a center frequency of the CPR signal.

Step 3: the terminal may determine a corresponding signal, in the first signal, within a range of 0.5 Hz centered on the center frequency of the CPR signal as the noise of the ECG signal caused by the CPR process.

It may be understood that the terminal may determine a second frequency of the first signal from a power spectrum estimation diagram of the first signal according to a second preset value. Here, the second frequency may be a center frequency of the first signal. It should be noted that the center frequency of the first signal is a frequency at which a peak with the maximum amplitude in the power spectrum estimation diagram of the first signal is located.

The terminal may analyze, by at least the following three methods, whether the noise of the ECG signal correlates with the CPR signal. The methods are not limited here.

First method: if the frequency interval between the center frequency of the first signal and the center frequency of the CPR signal is smaller than a first threshold, the terminal may determine that the noise of the ECG signal correlates with the CPR signal. Here, the first threshold is controllable, and the value range of the first threshold may be [1, 2].

Specifically, the frequency interval $f$ between the center frequency of the first signal and the center frequency of the CPR signal may be:

$$f = \|\text{the center frequency } f_1 \text{ of the first signal - the center frequency } f_2 \text{ of the CPR signal}\| \tag{9}$$

That is, if the frequency interval $f$ is smaller than the first threshold, the terminal may determine that the noise of the ECG signal correlates with the CPR signal.

Second method: if the ratio of the noise of the ECG signal caused by the CPR process to the first signal is greater than a second threshold, the terminal may determine that the noise of the ECG signal correlates with the CPR signal, wherein the value range of the second threshold may be [0.35, 0.4].

Specifically, the ratio $\theta_1$ of the noise of the ECG signal caused by the CPR process to the first signal may be:

$$\theta_1 = \text{the sum of amplitudes of a power spectrum curve of the first signal within}$$
$$\text{the range ([the center frequency } f_1 \text{ of the first signal - 0.5 Hz, the center frequency}}$$
$$f_1 \text{ of the first signal } + 0.5 \text{ Hz]) / (the sum of amplitudes of the power spectrum}}$$
$$\text{linearity of the first signal within the range [0.5 Hz, 40 Hz])} \tag{10}$$

That is, if the ratio $\theta_1$ is greater than the second threshold, the terminal may determine that the noise of the ECG signal correlates with the CPR signal.

Third method: if the ratio of the noise of the ECG signal caused by the CPR process to the first signal is greater than a third threshold and the frequency interval between the center frequency of the CPR signal and the center frequency of the first signal is smaller than a fourth threshold, the terminal may determine that the noise of the ECG signal correlates with the CPR signal. Here, the value range of the third threshold may be [0.25, 0.3], and the value range of the fourth threshold may be [1, 2].

That is, if the frequency interval $f$ is smaller than the fourth threshold and the ratio $\theta_1$ is greater than the third threshold, the terminal may determine that the noise of the ECG signal correlates with the CPR signal.

That is, the terminal may ascertain whether the noise of the ECG signal correlates with the CPR signal, according to the frequency interval between the center frequency of the first signal and the center frequency of the CPR signal and/or the ratio of the noise of the ECG signal caused by the CPR process to the first signal.

S203, if the noise of the ECG signal correlates with the CPR signal, the terminal may determine, according to a degree of the correlation between the noise of the ECG signal and the CPR signal, a filtering method for the first signal.

In this embodiment of the present application, the terminal may measure the degree of the correlation between the noise of the ECG signal and the CPR signal by at least one method. For detailed explanation, reference is made to step S204.

Here, the method for filtering the first signal by the terminal may include but is not limited to the following two methods:

First filtering method: the terminal filters the first signal by means of a high-order adaptive filter to remove the noise of the ECG signal. Here, the order of the high-order adaptive filter may be 4 to 6.

Second filtering method: the terminal filters the first signal by means of a low-order adaptive filter to remove the noise of the ECG signal. Here, the order of the low-order adaptive filter may be 2 to 3.

Specifically, the terminal may determine the filtering method for the first signal depending on whether the degree of the correlation between the noise of the ECG signal and the CPR signal exceeds a first value. If the terminal determines that the degree of the correlation between the noise of the ECG signal and the CPR signal exceeds the first value, the terminal may determine to use the first filtering method for filtering the first signal. Otherwise, the terminal may determine to use the second filtering method for filtering the first signal.

It should be noted that, compared with the second filtering method, the first filtering method may filter more noise of the ECG signal, but may also remove part of useful signal (ECG signal) in the first signal. The capability of the second filtering method (low-order adaptive filter) to remove the noise of the ECG signal is lower than that of the first filtering method (high-order adaptive filter), but may retain more ECG signals (avoid filtering out too many ECG signals in the first signal).

S204, the terminal ascertains whether the degree of the correlation between the noise of the ECG signal and the CPR signal exceeds the first value.

Specifically, the method that the terminal determines whether the degree of the correlation between the noise of the ECG signal and the CPR signal exceeds the first value may comprise:

the terminal ascertains whether the difference between the ratio of the noise of the ECG signal caused by the CPR process to the first signal and the ratio of the first signal component of the CPR signal is smaller than a fifth threshold. Specifically, the ratio $\theta_2$ of the first signal component of the CPR signal may be:

$$\theta_2 = \text{the sum of amplitudes of a power spectrum curve of the CPR signal within}$$
$$\text{the range ([the center frequency } f_2 \text{ of the CPR signal - 0.5 Hz, the center frequency}$$
$$f_2 \text{ of the CPR signal } + \text{ 0.5 Hz]) / (the sum of amplitudes of the power spectrum}$$
$$\text{curve of the CPR signal within the range [0.5 Hz, 40 Hz])} \tag{11}$$

In summary, the difference $\theta$ between the ratio $\theta_1$ of the noise of the ECG signal caused by the CPR process to the first signal and the ratio $\theta_2$ of the first signal component of the CPR signal may be:

$$\theta = \left\| \theta_1 - \theta_2 \right\| \tag{12}$$

That is, the method that the terminal ascertains whether the degree of the correlation exceeds the first value may comprise: the method for comparing the magnitude relationship between the difference $\theta$ and the fifth threshold. It may be understood that the fifth threshold is controllable, and the terminal determines, by setting the fifth threshold, the filtering method suitable for filtering the first signal. It should be noted that the value range of the fifth threshold may be [0.25, 0.3].

S205, if the degree of the correlation between the noise of the ECG signal and the CPR signal exceeds the first value, the terminal determines to use a first filtering method for filtering the first signal to remove the noise of the ECG signal.

Specifically, with reference to step S204, if the difference between the ratio of the noise of the ECG signal caused by the CPR process to the first signal and the ratio of the first signal component of the CPR signal is smaller than the fifth threshold, the terminal may determine that the degree of the correlation between the noise of the ECG signal and the CPR signal exceeds the first value, and then the terminal may determine to use the first filtering method for filtering the first signal to remove the noise of the ECG signal.

S206, if the degree of the correlation between the noise of the ECG signal and the CPR signal does not exceed the first value, the terminal may determine to use a second filtering method for filtering the first signal to remove the noise of the ECG signal.

Specifically, with reference to step S204, if the difference between the ratio of the noise of the ECG signal caused by the CPR process to the first signal and the ratio of the first signal component of the CPR signal is not smaller than the fifth threshold, the terminal may determine that the degree of the correlation between the noise of the ECG signal and the CPR signal does not exceed the first value, and then the terminal may determine to use the second filtering method for filtering the first signal to remove the noise of the ECG signal.

It may be understood that, for related definitions and descriptions not provided in the method embodiment of Fig. 7, reference may be made to those in the method embodiment of Fig. 4, and details are not described herein again.

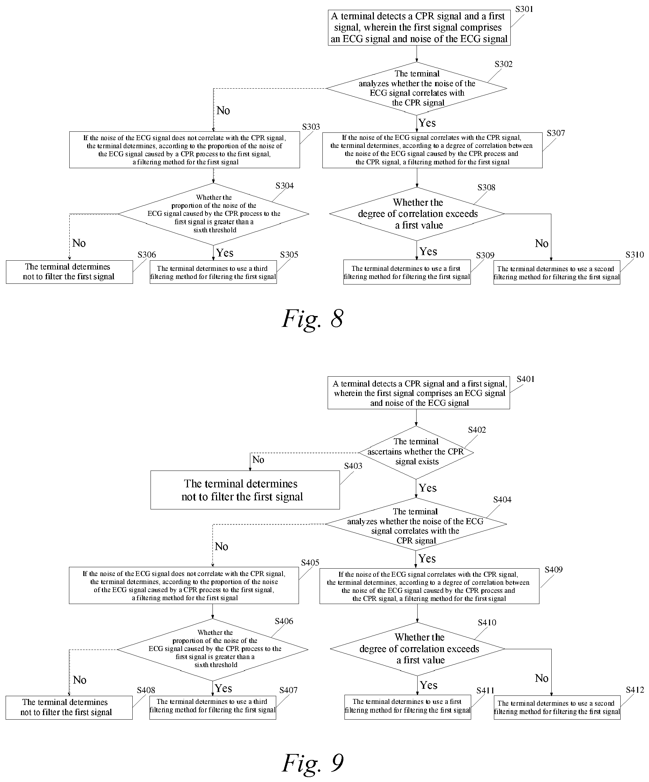

Fig. 8 is a schematic flowchart of still another ECG signal denoising method provided by the present application. In the embodiment of Fig. 8, if noise of an ECG signal does not correlate with a CPR signal, a terminal may determine, according to the ratio of the noise of the ECG signal to a first signal, a filtering method for the first signal. As shown in Fig. 8, the method may comprise at least the following steps:

S301, a terminal may detect a CPR signal and a first signal, wherein the first signal may comprise an ECG signal and noise of the ECG signal.

S302, the terminal may analyze whether the noise of the ECG signal in the first signal correlates with the CPR signal.

S303, if the noise of the ECG signal does not correlate with the CPR signal, the terminal may determine, according to the ratio of the noise of the ECG signal to the first signal, a filtering method for the first signal.

Specifically, the terminal may determine, according to the ratio of the noise of the ECG signal caused by the CPR process to the first signal, the filtering method for the first signal, which comprises:

a third filtering method: the terminal may filter the first signal by means of a high-order adaptive filter to remove the noise of the ECG signal. Here, the order of the high-order adaptive filter may be 4 to 6.

S304, the terminal may ascertain whether the ratio of the noise of the ECG signal caused by the CPR process to the first signal is greater than a sixth threshold.

It may be understood that the sixth threshold is controllable, and the terminal may determine the filtering method suitable for the first signal by setting the sixth threshold. It should be noted that the value range of the sixth threshold may be [0.2, 0.25]. Here, the noise of the ECG signal mainly arises from other factors (other than the CPR compression signal) in the surrounding environment.

S305, if the ratio of the noise of the ECG signal caused by the CPR process to the first signal is greater than the sixth threshold, the terminal may determine to use a third filtering method for filtering the first signal to remove the noise of the ECG signal.

Specifically, the capability of the third filtering method to suppress the noise of the ECG signal is higher than a fourth preset value. The terminal may illustrate with the fourth preset value that the third filtering method has a strong capability to suppress the noise of the ECG signal. It should be noted that, the filtering method (e.g., adaptive filter) having a higher order has a higher capability to remove noise. Here, the fourth preset value corresponds to a low-order filtering method. The capability of the third filtering method being higher than the fourth preset value means that the third filtering method is a high-order filtering method. For example, if the order of a low-order filtering method is [2, 3], the order of the third filtering method may be [4, 5]. If the order of the low-order filtering method is [4, 5], the order of the third filtering method is [6, 7].

S306, if the ratio of the noise of the ECG signal caused by the CPR process to the first signal is smaller than or equal to the sixth threshold (that is, the noise of the ECG signal in the first signal is negligible), the terminal may determine not to filter the first signal.

Specifically, if the noise in the first signal is low enough to be negligible, the terminal may not filter the first signal.

S307, if the noise of the ECG signal correlates with the CPR signal, the terminal may determine, according to a degree of the correlation between the noise of the ECG signal and the CPR signal, a filtering method for the first signal.

S308, the terminal may ascertain whether the degree of the correlation between the noise of the ECG signal and the CPR signal exceeds a first value.

S309, if the degree of the correlation between the noise of the ECG signal and the CPR signal exceeds the first value, the terminal may determine to use a first filtering method for filtering the first signal to remove the noise of the ECG signal.

S310, if the degree of the correlation between the noise of the ECG signal and the CPR signal does not exceed the first value, the terminal may determine to use a second filtering method for filtering the first signal to remove the noise of the ECG signal.

It may be understood that, for related definitions and descriptions not provided in the method embodiment of Fig. 8, reference may be made to those in the method embodiments of Fig. 4 and Fig. 7, and details are not described herein again.

Fig. 9 is a schematic flowchart of still another ECG signal denoising method provided by the present application. In the embodiment of Fig. 9, the terminal ascertains whether a CPR signal exists, and if the CPR signal does not exist, the terminal may not filter an ECG signal. As shown in Fig. 9, the method may comprise at least the following steps:

S401, a terminal detects a CPR signal and a first signal, wherein the first signal may comprise an ECG signal and noise of the ECG signal.

S402, the terminal may ascertain whether the CPR signal exists.

Specifically, after the terminal performs frequency domain analysis (for example, power spectrum estimation) on the CPR signal, the terminal may ascertain whether the number of peaks of the CPR signal in frequency domain is zero, and if the number of peaks is not zero, the terminal may determine that the CPR signal exists. Otherwise, the terminal may determine that the CPR signal does not exist.

S403, if the terminal determines that the CPR signal does not exist, the terminal may not filter the first signal.

Specifically, the terminal has not monitored or detected, by means of a CPR monitor, any CPR signal from a CPR sensor placed on a user's body (that is, if the first signal does not contain noise caused by a CPR process, the noise of the ECG signal is negligible). Then, the terminal may determine not to filter the first signal. It may be understood that, in the case that the noise of the ECG signal is negligible, if the terminal filters the first signal, it may remove too many ECG signals (pure signals) in the first signal, which causes serious distortion of the ECG signals.

S404, if the CPR signal exists, the terminal may analyze whether the noise of the ECG signal correlates with the CPR signal.

S405, if the noise of the ECG signal does not correlate with the CPR signal, the terminal may determine, according to the ratio of the noise of the ECG signal caused by the CPR process to the first signal, a filtering method for the first signal.

S406, the terminal may ascertain whether the ratio of the noise of the ECG signal caused by the CPR process to the first signal is greater than a sixth threshold.

S407, if the ratio of the noise of the ECG signal caused by the CPR process to the first signal is greater than the sixth threshold, the terminal may determine to use a third filtering method for filtering the first signal to remove the noise of the ECG signal.

S408, if the ratio of the noise of the ECG signal caused by the CPR process to the first signal is smaller than or equal to the sixth threshold (that is, the noise of the ECG signal in the first signal is negligible), the terminal may determine not to filter the first signal.

S409, if the noise of the ECG signal correlates with the CPR signal, the terminal may determine, according to a degree of the correlation between the noise of the ECG signal and the CPR signal, a filtering method for the first signal.

S410, the terminal may ascertain whether the degree of the correlation between the noise of the ECG signal and the CPR signal exceeds a first value.

S411, if the degree of the correlation between the noise of the ECG signal and the CPR signal exceeds the first value, the terminal may determine to use a first filtering method for filtering the first signal to remove the noise of the ECG signal.

S412, if the degree of the correlation between the noise of the ECG signal and the CPR signal does not exceed the first value, the terminal may determine to use a second filtering method for filtering the first signal to remove the noise of the ECG signal.

It may be understood that, for related definitions and descriptions not provided in the method embodiment of Fig. 9, reference may be made to those in the method embodiments of Fig. 4, Fig. 7 and Fig. 8, and details are not described herein again.

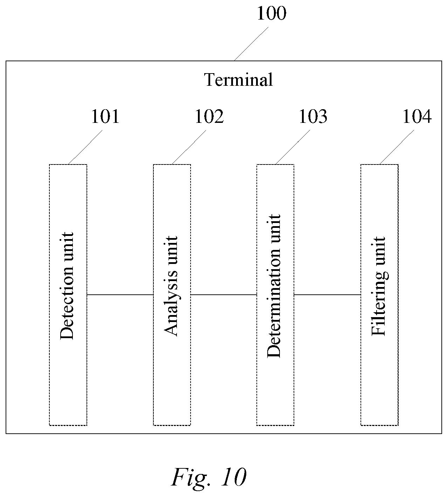

In order to facilitate the implementation of the embodiments of the present application, the present application provides a terminal for implementing the method described in the embodiment of Fig. 4, 7, 8 or 9. The terminal shown in Fig. 10 may be configured to perform the description in the corresponding embodiments described in all of the foregoing. As shown in Fig. 10, the terminal 100 comprises : a detection unit 101, an analysis unit 102, a determination unit 103, and a filtering unit 104.

The detection unit 101 is configured to detect a CPR signal and a first signal, wherein the first signal comprises an ECG signal and noise of the ECG signal.

The analysis unit 102 is configured to analyze whether the noise of the ECG signal in the first signal correlates with the CPR signal.

The determination unit 103 is configured to, if the analysis unit analyzes that the noise of the ECG signal correlates with the CPR signal, determine, according to a degree of the correlation between the noise of the ECG signal and the CPR signal, a filtering method for the first signal.

The filtering unit 104 is configured to filter the first signal according to the determined filtering method to remove the noise of the ECG signal.

The analysis unit 102 comprises a first determination unit, a second determination unit, and a third determination unit.

The first determination unit may be configured to determine a first frequency of the CPR signal, wherein the amplitude of the CPR signal at the first frequency exceeds a first preset value, and the first frequency is used to determine a frequency domain range in which the noise of the ECG signal is located.

The second determination unit may be configured to determine a second frequency of the first signal, wherein the amplitude of the first signal at the second frequency exceeds a second preset value.

The third determination unit may be configured to determine whether the noise of the ECG signal correlates with the CPR signal, according to the ratio of the noise of the ECG signal caused by the CPR process to the first signal and/or the frequency interval between the first frequency and the second frequency.

The third determination unit is specifically configured to:

determine, by at least the following three methods, whether the noise of the ECG signal correlates with the CPR signal.

First method: if the frequency interval between the center frequency of the first signal and the center frequency of the CPR signal is smaller than a first threshold, it may be determined that the noise of the ECG signal correlates with the CPR signal.

Second method: if the ratio of the noise of the ECG signal caused by the CPR process to the first signal is greater than a second threshold, it may be determined that the noise of the ECG signal correlates with the CPR signal.

Third method: if the ratio of the noise of the ECG signal caused by the CPR process to the first signal is greater than a third threshold and the frequency interval between the center frequency of the CPR signal and the center frequency of the first signal is smaller than a fourth threshold, it may be determined that the noise of the ECG signal correlates with the CPR signal.

The determination unit 103 may be specifically configured to:

if it is determined that the degree of the correlation exceeds a first value, determine to use a first filtering method for filtering the first signal, or otherwise, determine to use a second filtering method for filtering the first signal, wherein the order of the first filtering method is higher than the order of the second filtering method.

Further, if the difference between the ratio of the noise of the ECG signal caused by a CPR process to the first signal and the ratio of a first signal component of the CPR signal is smaller than a fifth threshold, the terminal determines that the degree of the correlation exceeds the first value, wherein the amplitude of the first signal component exceeds a third preset value.

The determination unit 103 may be further configured to, if the noise of the ECG signal does not correlate with the CPR signal, determine, according to the ratio of the noise of the ECG signal caused by the CPR process to the first signal, a filtering method for the first signal.

Specifically, if the ratio of the noise of the ECG signal caused by the CPR process to the first signal is greater than a sixth threshold, it is determined to use a third filtering method for filtering the first signal, wherein the capability of the third filtering method to suppress the noise of the ECG signal is higher than a fourth preset value.

Further, the terminal 100 may further comprise: in addition to the detection unit 101, the analysis unit 102, the determination unit 103, and the filtering unit 104: an ascertainment unit.

The ascertainment unit may be configured to ascertain whether the CPR signal exists, and perform, if the CPR signal exists, the step of the analysis unit 102 analyzing whether the noise of the ECG signal in the first signal correlates with the CPR signal.

The ascertainment unit may be specifically configured to:

after the analysis unit performs frequency domain analysis (power spectrum estimation) on the CPR signal, ascertain whether the number of peaks of the CPR signal in frequency domain is zero, and if the number is not zero, determine that the CPR signal exists, or otherwise, determine that the CPR signal does not exist.

It should be understood that the terminal 100 is only an example provided by the embodiment of the present application, and the terminal 100 may have more or fewer components than those shown, may combine two or more components, or may have different configurations of components.

It may be understood that, for specific implementations of the functional modules comprised in the terminal 100 of Fig. 10, reference may be made to the method embodiment of Fig. 4, 7, 8 or 9, and details are not described herein again.

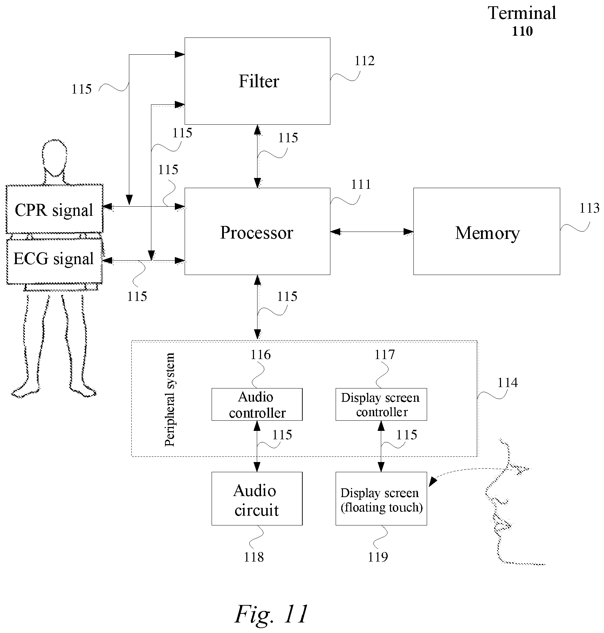

Fig. 11 is a schematic structural diagram of another terminal provided by the present application. In this embodiment of the present application, the terminal may comprise various medical apparatuses such as an electrocardiograph monitor, an electrocardiograph diagnostic instrument, an electrocardiograph instrument, or a temporary pacemaker, which is not limited in this embodiment of the present application. As shown in Fig. 11, the terminal 110 may comprise: one or more processors 111, one or more filters 112, a memory 113 (one or more computer-readable storage media), and a peripheral system 114. These components may communicate on one or more communication buses 115.

The memory 113 is coupled to the processor 111, and may be configured to store various software programs and/or multiple groups of instructions. In specific implementations, the memory 113 may comprise a high-speed random access memory, and may also comprise a non-volatile memory, for example, one or more magnetic disk storage apparatuses, flash memory apparatuses, or other non-volatile solid-state storage apparatuses. The memory 113 may store an operating system (hereinafter referred to as a system), for example, ANDROID, IOS, WINDOWS, or an embedded operating system such as LINUX. The memory 113 may also store a network communication program, which may be used to communicate with one or more additional apparatuses, one or more terminal apparatuses, and one or more network apparatuses. The memory 113 may also store a user interface program, which may display the content of an application program vividly by means of a graphical operation interface, and receive a user's control operations on the application program by means of input controls such as menus, dialog boxes, and keys.

It may be understood that the memory 113 may also be configured to store a first signal and a CPR signal. Here, the first signal may comprise an ECG signal and noise of the ECG signal.

It may be understood that the processor 111 may be configured to analyze the the correlation between the CPR signal and the noise of the ECG signal and the degree of the correlation between the noise of the ECG signal and the CPR signal, and determine a filtering method for the first signal. That is, the processor 111 may be configured to determine a filtering method (filter) suitable for filtering the first signal from the filter 112.

It may be understood that the filter 112 may be configured to filter the first signal to remove the noise of the ECG signal. It should be noted that the filter 112 may be one or more hardware filters integrated outside the processor 111, or one or more software programs that filter the first signal.

The peripheral system 114 is mainly configured to realize an interactive function between the terminal 110 and a user/external environment, and mainly comprises input and output devices of the terminal 100. In a specific implementation, the peripheral system 114 may comprise: an audio controller 116 and a display screen controller 117. Various controllers may be coupled to their respective peripheral apparatuses (for example, an audio circuit 118 and a display screen 119). In some embodiments, the display screen may be a display screen configured with a selfcapacitive floating touch panel, or a display screen configured with an infrared floating touch panel. It should be noted that the peripheral system 114 may further comprise other I/O peripherals.

It may be understood that the display screen 119 may be used by a doctor to observe a patient's ECG signal in real time, which facilitates the doctor in adjusting first-aid measures in time to rescue the patient. The audio circuit 118 may be configured to prompt the doctor in real time about the patient's physical state (the pulsating state of the patient's heart), and further output prompts suggesting that the doctor quickly adjusts the first-aid measures.

It should be understood that the terminal 110 is only an example provided by the embodiment of the present application, and the terminal 110 may have more or fewer components than those shown, may combine two or more components, or may have different configurations of components.

It may be understood that, for specific implementations of the functional modules comprised in the terminal 110 of Fig. 11, reference may be made to the method embodiment of Fig. 4, 7, 8 or 9, and details are not described herein again.

The present application provides a computer-readable storage medium, storing a computer program, which is implemented when executed by a processor.

The computer-readable storage medium may be an internal storage unit of the terminal described in any of the aforesaid embodiments, such as a hard disk or a memory of the terminal. The computer-readable storage medium may also be an external storage apparatus of the terminal, such as a removable hard disk, a smart media card (SMC), a secure digital (SD) card, or a flash card equipped on the terminal. Further, the computer-readable storage medium may further comprise both an internal storage unit and an external storage apparatus of the terminal. The computer-readable storage medium is used to store a computer program, and other programs and data required by the terminal. The computer-readable storage medium may also be used to temporarily store data that has been or will be output.

Those of ordinary skill in the art may realize that the units and algorithm steps of the various examples described in conjunction with the embodiments disclosed herein can be implemented in electronic hardware, computer software or a combination of both. In order to clearly illustrate hardware and software interchangeability, the compositions and steps of the various examples have been generally described in terms of function in the above description. Whether these functions are performed in hardware or software depends on the specific application and design constraints of the technical solution. Those skilled in the art could use different methods to implement the described functions for each particular application, but such implementation should not be considered to be beyond the scope of the present application.

Those skilled in the art would have clearly understood that for convenience and conciseness of description, the specific working processes of the above-described terminals and units can refer to the corresponding processes in the aforesaid embodiments of the method and will not be further described here.

In several embodiments provided in the present application, it is to be understood that the disclosed terminals and methods may be implemented in other ways, for example, to describe the compositions and steps of the various examples. Whether these functions are performed in hardware or software depends on the specific application and design constraints of the technical solution. Those skilled in the art could use different methods to implement the described functions for each particular application, but such implementation should not be considered to be beyond the scope of the present invention.

The terminal embodiments described above are merely exemplary. For example, the division of the units is only a logic function division. In actual implementation, there may be other division methods, for example, multiple units or components may be combined or integrated into another system, or some features may be omitted or not implemented. In addition, the mutual coupling or direct coupling or communication connection shown or discussed may be indirect coupling or communication connection through some interfaces, terminal or units, and may be in electrical, mechanical or other forms.

The units described as separate parts may or may not be physically separated, and the parts displayed as units may or may not be physical units, i.e., may be located in one place or may be distributed over multiple network units. Some or all of the units can be selected according to actual needs to achieve the purpose of solutions of the embodiments of the present application.

In addition, the functional units in the embodiments of the present application may be integrated into one processing unit or may alternatively exist as being physically separate, or two or more of the units may be integrated into one unit. The above integrated unit can be implemented in the form of hardware or a software function unit.

If the integrated unit is implemented in the form of a software function unit and sold or used as an independent product, it may be stored in a computer-readable storage medium. Based on such an understanding, the technical solutions of the present application essentially, or the part contributing to the prior art may be implemented in the form of a software product. The computer software product may be stored in a storage medium and comprises several instructions for instructing a computer apparatus (which may be a personal computer, a server, or a network apparatus) to perform all or some of the steps of the methods described in the embodiments of the present application. The aforesaid storage medium comprises: a U disk, a removable hard disk, read-only memory (ROM), a random access memory (RAM), a magnetic disk or an optical disk and other media that can store program codes.

The above descriptions are merely the specific embodiments of the present application, but the scope of protection of the present application is not limited thereto, those skilled in the art would readily think of various equivalent modifications or substitutions within the technical scope disclosed in the present application, and these modifications or substitutions should all be intended to be included within the scope of protection of the present application. Therefore, the scope of protection of the present application is defined by the claims.

## Claims

1. A computer program for denoising an ECG signal comprising instructions to cause a terminal as defined in claim 9 to perform the steps comprising:

   detecting (S101, S201, S301, S401) a CPR signal and a first signal wherein the first signal comprises an ECG signal and a noise of the ECG signal;
   analyzing (S102, S202, S302, S404) whether the noise of the ECG signal in the first signal correlates with the CPR signal;
   determining (S103, S203, S307, S409) a filtering method for the first signal according to a degree of the correlation between the noise of the ECG signal and the CPR signal, when the noise of the ECG signal correlates with the CPR signal; further determining (S205, S309, S411) a first filtering method for the first signal, when the degree of the correlation is ascertained to exceed a first value (S204, S308, S410), otherwise, determining (S206, S310, S412) a second filtering method for the first signal; through the first filtering method, the first signal is filtered by a high-order adaptive filter to remove the noise of the ECG signal; through the second filtering method, the first signal is filtered by a low-order adaptive filter to remove the noise of the ECG signal; wherein an order of the high-order adaptive filter is higher than an order of the low-order adaptive filter; and
   filtering (S104) the first signal according to the determined filtering method to remove the noise of the ECG signal.

2. The computer program of claim 1, **characterized in that** the step of analyzing whether the noise of the ECG signal in the first signal correlates with the CPR signal specifically comprises:

   determining a first frequency of the CPR signal, wherein an amplitude of the CPR signal at the first frequency exceeds a first preset value, and the first frequency is used to determine a frequency domain range in which the noise of the ECG signal is located;
   determining a second frequency of the first signal, wherein an amplitude of the first signal at the second frequency exceeds a second preset value; and
   determining whether the noise of the ECG signal correlates with the CPR signal, according to a ratio of the noise of the ECG signal caused by the CPR process to the first signal, and/or, according to a frequency interval between the first frequency and the second frequency.

3. The computer program of claim 2, **characterized in that** the step of determining whether the noise of the ECG signal correlates with the CPR signal, according to a ratio of the noise of the ECG signal caused by the CPR process to the first signal, and/or, according to a frequency interval between the first frequency and the second frequency specifically comprises:

determining that the noise of the ECG signal correlates with the CPR signal when the frequency interval between the first frequency and the second frequency is smaller than a first threshold; or

determining that the noise of the ECG signal correlates with the CPR signal when the ratio is greater than a second threshold; or

determining that the noise of the ECG signal correlates with the CPR signal when the ratio is greater than a third threshold and the frequency interval between the first frequency and the second frequency is smaller than a fourth threshold.

4. The computer program of claim 1, **characterized in that** the step of the degree of the correlation is ascertained to exceed a first value specifically comprises:
determining that the degree of the correlation exceeds the first value, when a difference between a ratio of the noise of the ECG signal to the first signal and a ratio of a first signal component of the CPR signal to the CPR signal is smaller than a fifth threshold, wherein an amplitude of the first signal component exceeds a third preset value.

5. The computer program of claim 1, further comprising:
ascertaining whether the CPR signal exists, and performing the step of analyzing whether the noise of the ECG signal in the first signal correlates with the CPR signal, when the CPR signal exists.

6. The computer program of claim 5, **characterized in that** ascertaining, by the terminal, whether the CPR signal exists specifically comprises:
ascertaining, by the terminal, whether a number of peaks of the CPR signal in a frequency domain is zero, after the terminal performs a frequency domain analysis (a power spectrum estimation) on the CPR signal; determining, by the terminal, that the CPR signal exists, when the number is not zero; and determining, by the terminal, that the CPR signal does not exist, when the number is zero.

7. The computer program of claim 1, further comprising:
determining a filtering method for the first signal, according to a ratio of the noise of the ECG signal caused by a CPR process to the first signal, when the noise of the ECG signal does not correlate with the CPR signal.

8. The computer program of claim 7, wherein the step of determining (S303, S405) a filtering method for the first signal, according to a ratio of the noise of the ECG signal caused by a CPR process to the first signal, when the noise of the ECG signal does not correlate with the CPR signal, specifically comprises:
determining (S304, S406) a third filtering method for the first signal, when the ratio of the noise of the ECG signal caused by the CPR process to the first signal is greater than a sixth threshold, wherein capability of the third filtering method to suppress the noise of the ECG signal is higher than a fourth preset value.

9. A terminal, comprising:

a detection unit (101) configured to detect a CPR signal and a first signal, wherein the first signal comprises an ECG signal and a noise of the ECG signal;

an analysis unit (102) configured to analyze whether the noise of the ECG signal in the first signal correlates with the CPR signal;

a determination unit (103) configured to determine a filtering method for the first signal according to a degree of the correlation between the noise of the ECG signal and the CPR signal, when the analysis unit (102) analyzes that the noise of the ECG signal correlates with the CPR signal; and

a filtering unit (104) configured to filter the first signal according to the determined filtering method to remove the noise of the ECG signal, **characterised in**
the filtering method comprising: a first filtering method, through which the first signal is filtered by a high-order adaptive filter to remove the noise of the ECG signal; a second filtering method, through which the first signal is filtered by a low-order adaptive filter to remove the noise of the ECG signal; the determination unit (103) further configured to determine the first filtering method for the first signal, when the degree of the correlation exceeds a first value, otherwise, determine a second filtering method for the first signal, wherein an order of a filter in the first filtering method is higher than an order of a filter in the second filtering method.

10. The terminal of claim 9, **characterized in that** the analysis unit (102) comprises:

a first determination unit configured to determine a first frequency of the CPR signal, wherein an amplitude of the CPR signal at the first frequency exceeds a first preset value, and the first frequency is configured to determine a frequency domain range in which the noise of the ECG signal is located;
a second determination unit configured to determine a second frequency of the first signal, wherein an amplitude of the first signal at the second frequency exceeds a second preset value; and
a third determination unit configured to determine whether the noise of the ECG signal correlates with the CPR signal, according to a ratio of the noise of the ECG signal caused by the CPR process to the first signal, and/or, according to a frequency interval between the first frequency and the second frequency.

11. The terminal of claim 10, **characterized in that** the third determination unit is specifically configured to:

determine that the noise of the ECG signal correlates with the CPR signal when the frequency interval between the first frequency and the second frequency is smaller than a first threshold; or
determine that the noise of the ECG signal correlates with the CPR signal when the ratio is greater than a second threshold; or
determine that the noise of the ECG signal correlates with the CPR signal when the ratio is greater than a third threshold and the frequency interval between the first frequency and the second frequency is smaller than a fourth threshold.

12. The terminal of claim 9, further comprising:
an ascertainment unit configured to ascertain whether the CPR signal exists, and perform the step of the terminal analyzing whether the noise of the ECG signal in the first signal correlates with the CPR signal, when the CPR signal exists.

13. The terminal of claim 9, **characterized in that** the determination unit (103) is further configured to:
determine a filtering method for the first signal, according to a ratio of the noise of the ECG signal caused by a CPR process to the first signal, when the noise of the ECG signal does not correlate with the CPR signal.

14. The terminal of claim 9, **characterized in that** an order of the high-order adaptive filter may be selected from 4 to 6, and an order of the low-order adaptive filter may be selected from 2 to 3.

15. A computer-readable storage medium, storing a computer program in accordance with claim 1.

**Patentansprüche**

1. Ein Computerprogramm zum Entrauschen eines EKG-Signals mit Anweisungen für ein Endgerät gemäß Anspruch 9, die folgenden Schritte auszuführen:

die Erfassung (S101, S201, S301, S401) eines CPR-Signals und eines ersten Signals, wobei das erste Signal ein EKG-Signal und ein Rauschen des EKG-Signals enthält,
die Analyse (S102, S202, S302, S404), ob das Rauschen des EKG-Signals im ersten Signal mit dem CPR-Signal korreliert,
die Festlegung (S103, S203, S307, S409) eines Filterverfahrens für das erste Signal anhand eines Korrelationsgrads zwischen dem Rauschen des EKG-Signals und dem CPR-Signal, wenn das Rauschen des EKG-Signals mit dem CPR-Signal korreliert, weiterhin die Festlegung (S205, S309, S411) eines ersten Filterverfahrens für das erste Signal, wenn der Korrelationsgrad nachweislich einen ersten Wert überschreitet (S204, S308, S410), anderenfalls die Festlegung (S206, S310, S412) eines zweiten Filterverfahrens für das erste Signal, wobei das erste Signal im ersten Filterverfahren über einen adaptiven Filter höherer Ordnung gefiltert wird, um Rauschen aus dem EKG-Signal zu entfernen und das erste Signal im zweiten Filterverfahren über einen adaptiven Filter niedriger Ordnung gefiltert wird, um das Rauschen aus dem EKG-Signal zu entfernen, wobei eine Ordnung des adaptiven Filters höherer Ordnung höher ist als eine Ordnung des adaptiven Filters niedriger Ordnung, und
das Filtern (S104) des ersten Signals anhand des festgelegten Filterverfahrens zur Entfernung des Rauschens aus dem EKG-Signal.

2. Das Computerprogramm gemäß Anspruch 1, **dadurch gekennzeichnet, dass** der Analyseschritt, ob das Rauschen des EKG-Signals im ersten Signal mit dem CPR-Signal korreliert insbesondere Folgendes umfasst:

die Bestimmung einer ersten Frequenz des CPR-Signals, wobei eine Amplitude des CPR-Signals bei der ersten Frequenz einen vorbestimmten Wert überschreitet und die erste Frequenz zur Bestimmung eines Frequenzbereichs verwendet wird, in dem das Rauschen des EKG-Signals gelegen ist,
die Bestimmung einer zweiten Frequenz des ersten Signals, wobei die Amplitude des ersten Signals auf der zweiten Frequenz einen zweiten vorbestimmten Wert überschreitet, und
die Bestimmung, ob das Rauschen des EKG-Signals mit dem CPR-Signal korreliert und zwar anhand eines Verhältnisses des durch den CPR-Prozess beim ersten Signal hervorgerufenen Rauschens des EKG-Signals und/oder anhand eines Frequenzintervalls zwischen erster Frequenz und zweiter Frequenz.

3. Das Computerprogramm gemäß Anspruch 2, **dadurch gekennzeichnet, dass** der Schritt der Feststellung, ob das Rauschen des EKG-Signals mit dem CPR-Signal korreliert und zwar anhand eines Verhältnisses des durch den CPR-Prozess am ersten Signal hervorgerufenen Rauschens des EKG-Signals und/oder anhand eines Frequenzintervalls zwischen erster Frequenz und zweiter Frequenz insbesondere Folgendes umfasst:

die Feststellung, ob das Rauschen des EKG-Signals mit dem CPR-Signal korreliert, wenn das Frequenzintervall zwischen erster Frequenz und zweiter Frequenz kleiner als ein erster Schwellenwert ist, oder
die Feststellung, ob das Rauschen des EKG-Signals mit dem CPR-Signal korreliert, wenn das Verhältnis über einem zweiten Schwellenwert liegt, oder
die Feststellung, ob das Rauschen des EKG-Signals mit dem CPR-Signal korreliert, wenn das Verhältnis über einem dritten Schwellenwert liegt und das Frequenzintervall zwischen erster Frequenz und zweiter Frequenz kleiner als ein vierter Schwellenwert ist.

4. Das Computerprogramm gemäß Anspruch 1, **dadurch gekennzeichnet, dass** der Schritt des nachweislichen Überschreitens eines ersten Werts für den Korrelationsgrad insbesondere Folgendes umfasst:
die Feststellung, ob der Korrelationsgrad einen ersten Wert überschreitet, wenn die Differenz zwischen einem Verhältnis des Rauschens des EKG-Signals zum ersten Signal und ein Verhältnis eines ersten Signalbestandteils des CPR-Signals zum CPR-Signal kleiner als ein fünfter Schwellenwert ist, wobei eine Amplitude des ersten Signalbestandteils einen dritten voreingestellten Wert überschreitet.

5. Das Computerprogramm gemäß Anspruch 1, das weiterhin Folgendes aufweist:
den Nachweis, ob ein CPR-Signal vorhanden ist, und die Ausführung des Analyseschritts, ob das Rauschen des EKG-Signals im ersten Signal mit dem CPR-Signal korreliert, wenn das CPR-Signal vorhanden ist.

6. Das Computerprogramm gemäß Anspruch 5, **dadurch gekennzeichnet, dass** der Nachweis durch das Endgerät, ob ein CPR-Signal vorhanden ist, insbesondere Folgendes umfasst:
den Nachweis durch das Endgerät, ob eine Anzahl von Peaks des CPR-Signals in einem Frequenzbereich bei Null liegt, nachdem das Endgerät eine Frequenzbereichsanalyse (Schätzung des Leistungsspektrums) für das CPR-Signal durchgeführt hat, die Feststellung durch das Endgerät, dass das CPR-Signal vorhanden ist, wenn die Anzahl nicht gleich Null ist, und die Feststellung durch das Endgerät, dass das CPR-Signal nicht vorhanden ist, wenn die Anzahl Null entspricht.

7. Das Computerprogramm gemäß Anspruch 1, das weiterhin Folgendes aufweist:
die Festlegung eines Filterverfahrens für das erste Signal anhand eines Verhältnisses des durch den CPR-Prozess verursachten Rauschens des ersten Signals, wenn das Rauschen des EKG-Signals nicht mit dem CPR-Signal korreliert.

8. Das Computerprogramm gemäß Anspruch 7, wobei der Schritt der Festlegung (S303, S405) eines Filterverfahrens für das erste Signal anhand eines Verhältnisses des durch den CPR-Prozess verursachten Rauschens des ersten Signals, wenn das Rauschen des EKG-Signals nicht mit dem CPR-Signal korreliert, insbesondere Folgendes umfasst:
die Festlegung (S304, S406) eines dritten Filterverfahrens für das erste Signal, wenn das Verhältnis des durch den CPR-Prozess verursachten Rauschens des ersten Signals über einem sechsten Schwellenwert liegt, wobei die Fähigkeit des dritten Filterverfahrens zur Unterdrückung des Rauschens des EKG-Signals über einem vierten vorbestimmten Wert liegt.

9. Ein Endgerät mit:

einer Erfassungseinheit (101), die zur Erfassung eines CPR-Signals und eines ersten Signals ausgelegt ist, wobei das erste Signal ein EKG-Signal und ein Rauschen des EKG-Signals enthält,
einer Analyseeinheit (102), die zur Analyse ausgelegt ist, ob das Rauschen des EKG-Signals im ersten Signal mit dem CPR-Signal korreliert,
einer Festlegungseinheit (103), die zur Festlegung eines Filterverfahrens für das erste Signal anhand eines Korrelationsgrads zwischen dem Rauschen des EKG-Signals und dem CPR-Signal ausgelegt ist, wenn die Analyseeinheit (102) feststellt, dass das Rauschen des EKG-Signals mit dem CPR-Signal korreliert,
einer Filtereinheit (104), die zum Filtern des ersten Signals anhand des festgelegten Filterverfahrens zur Entfernung des Rauschens aus dem EKG-Signal ausgelegt ist,
**dadurch gekennzeichnet, dass** das Filterverfahren Folgendes umfasst: ein erstes Filterverfahren, mit dem das erste Verfahren über einen adaptiven Filter höherer Ordnung gefiltert wird, um das Rauschen des EKG-Signals zu entfernen, ein zweites Filterverfahren, mit dem das erste Signal über einen adaptiven Filter niedriger Ordnung gefiltert wird, um das Rauschen des EKG-Signals zu entfernen, wobei die Festlegungseinheit (103) weiterhin so ausgelegt ist, dass sie das erste Filterverfahren für das erste Signal festlegt, wenn der Korrelationsgrad einen ersten Wert überschreitet, und anderenfalls ein zweites Filterverfahren für das erste Signal festlegt, wobei eine Ordnung des Filters des ersten Filterverfahrens höher ist als eine Ordnung des Filters im zweiten Filterverfahren.

10. Das Endgerät gemäß Anspruch 9, **dadurch gekennzeichnet, dass** die Analyseeinheit (102) Folgendes umfasst:

eine erste Bestimmungseinheit, die zur Bestimmung einer ersten Frequenz des CPR-Signals ausgelegt ist, wobei eine Amplitude des CPR-Signals bei der ersten Frequenz einen vorbestimmten Wert überschreitet und die erste Frequenz zur Bestimmung eines Frequenzbereichs ausgelegt ist, in dem das Rauschen des EKG-Signals gelegen ist,
eine zweite Bestimmungseinheit zur Bestimmung einer zweiten Frequenz des ersten Signals, wobei die Amplitude des ersten Signals auf der zweiten Frequenz einen zweiten vorbestimmten Wert überschreitet, und
einer dritten Bestimmungseinheit zur Feststellung, ob das Rauschen des EKG-Signals mit dem CPR-Signal korreliert und zwar anhand eines Verhältnisses des durch den CPR-Prozess am ersten Signal hervorgerufenen Rauschens des EKG-Signals und/oder anhand eines Frequenzintervalls zwischen erster Frequenz und zweiter Frequenz.

11. Das Endgerät gemäß Anspruch 10, **dadurch gekennzeichnet, dass** die dritte Bestimmungseinheit insbesondere für Folgendes ausgelegt ist:

die Feststellung, ob das Rauschen des EKG-Signals mit dem CPR-Signal korreliert, wenn das Frequenzintervall zwischen erster Frequenz und zweiter Frequenz kleiner als ein erster Schwellenwert ist, oder
die Feststellung, ob das Rauschen des EKG-Signals mit dem CPR-Signal korreliert, wenn das Verhältnis über einem zweiten Schwellenwert liegt, oder
die Feststellung, ob das Rauschen des EKG-Signals mit dem CPR-Signal korreliert, wenn das Verhältnis über einem dritten Schwellenwert liegt und das Frequenzintervall zwischen erster Frequenz und zweiter Frequenz kleiner als ein vierter Schwellenwert ist.

12. Das Endgerät gemäß Anspruch 9, das weiterhin Folgendes aufweist:
eine Nachweiseinheit, die für den Nachweis ausgelegt ist, ob ein CPR-Signal vorhanden ist, und die Ausführung des Analyseschritts des Endgeräts, ob das Rauschen des EKG-Signals im ersten Signal mit dem CPR-Signal korreliert, wenn das CPR-Signal vorhanden ist.

13. Das Endgerät gemäß Anspruch 9, **dadurch gekennzeichnet, dass** die Festlegungseinheit (103) weiterhin ausgelegt ist:
zur Festlegung eines Filterverfahrens für das erste Signal anhand eines Verhältnisses des durch den CPR-Prozess verursachten Rauschens des ersten Signals, wenn das Rauschen des EKG-Signals nicht mit dem CPR-Signal korreliert.

14. Das Endgerät gemäß Anspruch 9, **dadurch gekennzeichnet, dass** eine Ordnung des adaptiven Filters höherer Ordnung zwischen 4 und 6 gewählt werden kann und eine Ordnung des adaptiven Filters niedriger Ordnung zwischen 2 und 3 gewählt werden kann.

**15.** Ein computerlesbares Speichermedium zur Speicherung des Computerprogramms gemäß Anspruch 1.


**Revendications**

**1.** Programme informatique de débruitage d'un signal d'électrocardiogramme (ECG) comprenant des instructions pour amener un terminal selon la revendication 9 à exécuter les étapes comprenant :

détecter (S101, S201, S301, S401) un signal de réanimation cardiopulmonaire (RCP) et un premier signal où le premier signal comprend un signal d'ECG et un bruit du signal d'ECG ;
analyser (S102, S202, S302, S404) si le bruit du signal d'ECG dans le premier signal est corrélé au signal de RCP ;
déterminer (S103, S203, S307, S409) une méthode de filtrage pour le premier signal selon un degré de la corrélation entre le bruit du signal d'ECG et le signal de RCP, lorsque le bruit du signal d'ECG est corrélé au signal de RCP ; déterminer en outre (S205, S309, S411) un première méthode de filtrage pour le premier signal, lorsqu'il est vérifié que le degré de la corrélation dépasse une première valeur (S204, S308, S410), sinon, déterminer (S206, S310, S412) une deuxième méthode de filtrage pour le premier signal ; via la première méthode de filtrage, le premier signal est filtré par un filtre adaptatif d'ordre élevé pour éliminer le bruit du signal d'ECG ; via la deuxième méthode de filtrage, le premier signal est filtré par un filtre adaptatif de faible ordre pour éliminer le bruit du signal d'ECG ; où un ordre du filtre adaptatif d'ordre élevé est supérieur à un ordre du filtre adaptatif de faible ordre ; et
filtrer (S104) le premier signal selon la méthode de filtrage déterminée pour éliminer le bruit du signal d'ECG.

**2.** Programme informatique selon la revendication 1, **caractérisé en ce que** l'étape consistant à analyser si le bruit du signal d'ECG dans le premier signal est corrélé au signal de RCP comprend spécifiquement :

le fait de déterminer une première fréquence du signal de RCP, où une amplitude du signal de RCP à la première fréquence dépasse une première valeur préétablie, et la première fréquence est utilisée pour déterminer une plage de domaine de fréquences dans laquelle se situe le bruit du signal d'ECG ;
le fait de déterminer une deuxième fréquence du premier signal, où une amplitude du premier signal à la deuxième fréquence dépasse une deuxième valeur préétablie ; et
le fait de déterminer si le bruit du signal d'ECG est corrélé au signal de RCP, selon un rapport du bruit du signal d'ECG dû au processus de RCP sur le premier signal, et/ou selon un intervalle de fréquence entre la première fréquence et la deuxième fréquence.

**3.** Programme informatique selon la revendication 2, **caractérisé en ce que** l'étape consistant à déterminer si le bruit du signal d'ECG est corrélé au signal de RCP, selon un rapport du bruit du signal d'ECG dû au processus de RCP sur le premier signal, et/ou selon un intervalle de fréquence entre la première fréquence et la deuxième fréquence comprend spécifiquement :

le fait de déterminer que le bruit du signal d'ECG est corrélé au signal de RCP lorsque l'intervalle de fréquence entre la première fréquence et la deuxième fréquence est inférieur à un premier seuil ; ou
le fait de déterminer que le bruit du signal d'ECG est corrélé au signal de RCP lorsque le rapport est supérieur à un deuxième seuil ; ou
le fait de déterminer que le bruit du signal d'ECG est corrélé au signal de RCP lorsque le rapport est supérieur à un troisième seuil et que l'intervalle de fréquence entre la première fréquence et la deuxième fréquence est inférieur à un quatrième seuil.

**4.** Programme informatique selon la revendication 1, **caractérisé en ce que** l'étape consistant à vérifier que le degré de la corrélation dépasse une première valeur comprend spécifiquement :
le fait de déterminer que le degré de la corrélation dépasse la première valeur, lorsqu'une différence entre un rapport du bruit du signal d'ECG sur le premier signal et un rapport d'une composante de premier signal du signal de RCP sur le signal de RCP est inférieure à un cinquième seuil, où une amplitude de la composante de premier signal dépasse une troisième valeur préétablie.

**5.** Programme informatique selon la revendication 1, comprenant en outre :
le fait de vérifier si le signal de RCP existe, et d'effectuer l'étape consistant à analyser si le bruit du signal d'ECG dans le premier signal est corrélé au signal de RCP, lorsque le signal de RCP existe.

**6.** Programme informatique selon la revendication 5, **caractérisé en ce que** le fait de vérifier, par le terminal, si le signal de RCP existe comprend spécifiquement :
le fait de vérifier, par le terminal, si un nombre de pics du signal de RCP dans un domaine de fréquences est nul, après que le terminal a exécuté une analyse de domaine de fréquences (une estimation de spectre de puissance) sur le signal de RCP ; le fait de déterminer, par le terminal, que le signal de RCP existe, lorsque le nombre n'est pas nul ; et le fait de déterminer, par le terminal, que le signal de RCP n'existe pas, lorsque le nombre est nul.

**7.** Programme informatique selon la revendication 1, comprenant en outre :
le fait de déterminer une méthode de filtrage pour le premier signal, selon un rapport du bruit du signal d'ECG dû à un processus de RCP sur le premier signal, lorsque le bruit du signal d'ECG n'est pas corrélé au signal de RCP.

**8.** Programme informatique selon la revendication 7, dans lequel l'étape consistant à déterminer (S303, S405) une méthode de filtrage pour le premier signal, selon un rapport du bruit du signal d'ECG dû à un processus de RCP sur le premier signal, lorsque le bruit du signal d'ECG n'est pas corrélé au signal de RCP, comprend spécifiquement :
le fait de déterminer (S304, S406) une troisième méthode de filtrage pour le premier signal, lorsque le rapport du bruit du signal d'ECG dû au processus de RCP sur le premier signal est supérieur à un sixième seuil, où la capacité de la troisième méthode de filtrage pour supprimer le bruit du signal d'ECG est supérieure à une quatrième valeur préétablie.

**9.** Terminal, comprenant :

une unité de détection (101) configurée pour détecter un signal de RCP et un premier signal, où le premier signal comprend un signal d'ECG et un bruit du signal d'ECG ;
une unité d'analyse (102) configurée pour analyser si le bruit du signal d'ECG dans le premier signal est corrélé au signal de RCP ;
une unité de détermination (103) configurée pour déterminer une méthode de filtrage pour le premier signal selon un degré de la corrélation entre le bruit du signal d'ECG et le signal de RCP, lorsque l'unité d'analyse (102) analyse que le bruit du signal d'ECG est corrélé au signal de RCP ; et
une unité de filtrage (104) configurée pour filtrer le premier signal selon la méthode de filtrage déterminée pour éliminer le bruit du signal d'ECG, **caractérisé en ce que**
le procédé de filtrage comprenant : une première méthode de filtrage, par laquelle le premier signal est filtré par un filtre adaptatif d'ordre élevé pour éliminer le bruit du signal d'ECG ; une deuxième méthode de filtrage, par laquelle le premier signal est filtré par un filtre adaptatif de faible ordre pour éliminer le bruit du signal d'ECG ; l'unité de détermination (103) étant en outre configurée pour déterminer la première méthode de filtrage pour le premier signal, lorsque le degré de la corrélation dépasse une première valeur, sinon, déterminer une deuxième méthode de filtrage pour le premier signal, où un ordre d'un filtre dans la première méthode de filtrage est supérieur à un ordre d'un filtre dans la deuxième méthode de filtrage.

**10.** Terminal selon la revendication 9, **caractérisé en ce que** l'unité d'analyse (102) comprend :

une première unité de détermination configurée pour déterminer une première fréquence du signal de RCP, où une amplitude du signal de RCP à la première fréquence dépasse une première valeur préétablie, et la première fréquence est configurée pour déterminer une plage de domaine de fréquences dans laquelle se situe le bruit du signal d'ECG ;
une deuxième unité de détermination configurée pour déterminer une deuxième fréquence du premier signal, où une amplitude du premier signal à la deuxième fréquence dépasse une deuxième valeur préétablie ; et
une troisième unité de détermination configurée pour déterminer si le bruit du signal d'ECG est corrélé au signal de RCP, selon un rapport du bruit du signal d'ECG dû au processus de RCP sur le premier signal, et/ou selon un intervalle de fréquence entre la première fréquence et la deuxième fréquence.

**11.** Terminal selon la revendication 10, **caractérisé en ce que** la troisième unité de détermination est spécifiquement configurée pour :

déterminer que le bruit du signal d'ECG est corrélé au signal de RCP lorsque l'intervalle de fréquence entre la première fréquence et la deuxième fréquence est inférieur à un premier seuil ; ou
déterminer que le bruit du signal d'ECG est corrélé au signal de RCP lorsque le rapport est supérieur à un deuxième seuil ; ou
déterminer que le bruit du signal d'ECG est corrélé au signal de RCP lorsque le rapport est supérieur à un troisième seuil et que l'intervalle de fréquence entre la première fréquence et la deuxième fréquence est inférieur à un quatrième seuil.

12. Terminal selon la revendication 9, comprenant en outre :
une unité de vérification configurée pour vérifier si le signal de RCP existe, et effectuer l'étape consistant à analyser par le terminal si le bruit du signal d'ECG dans le premier signal est corrélé au signal de RCP, lorsque le signal de RCP existe.

13. Terminal selon la revendication 9, **caractérisé en ce que** l'unité de détermination (103) est en outre configurée pour :
déterminer une méthode de filtrage pour le premier signal, selon un rapport du bruit du signal d'ECG dû à un processus de RCP sur le premier signal, lorsque le bruit du signal d'ECG n'est pas corrélé au signal de RCP.

14. Terminal selon la revendication 9, **caractérisé en ce qu'**un ordre du filtre adaptatif d'ordre élevé peut être choisi entre 4 et 6, et un ordre du filtre adaptatif de faible ordre peut être choisi entre 2 et 3.

15. Support de stockage lisible par ordinateur, stockant un programme informatique selon la revendication 1.

Interference-free
original ECG signal

ECG signal after
low-order adaptive filtering

ECG signal after
high-order adaptive filtering

CPR compression signal

*Fig. 1*

Seriously interfered
original arrest signal

Signal after low-order
adaptive filtering

Signal after high-order
adaptive filtering

CPR compression signal

*Fig. 2*

ECG signal having a
relatively good correlation with
a CPR compression signal

ECG signal after
low-order adaptive filtering

ECG signal after
high-order adaptive filtering

CPR compression signal

*Fig. 3*

S101

A terminal detects a CPR signal and a first signal, wherein the first signal comprises an ECG signal and noise of the ECG signal

S102

The terminal analyzes whether the noise of the ECG signal in the first signal correlates with the CPR signal

S103

If the noise of the ECG signal correlates with the CPR signal, the terminal determines, according to a degree of correlation between the noise of the ECG signal and the CPR signal, a filtering method for the first signal

S104

The terminal filters the first signal according to the determined filtering method to filter out the noise of the ECG signal

*Fig. 4*

**x**(n) → Parameter-adjustable digital filter → y(n)

−

$d$(n) → ∑ → $e$(n)

+

Adaptive algorithm

*Fig. 5*

**x**(n) → $z^{-1}$ → $z^{-1}$ → ... → $z^{-1}$

$w_0$   $w_1$   $w_2$   $w_M$

+ → + → ... → + → y(n)

*Fig. 6*

A terminal detects a CPR signal and a first signal, wherein the first signal comprises an ECG signal and noise of the ECG signal — S201

The terminal analyzes whether the noise of the ECG signal in the first signal correlates with the CPR signal — S202

If the noise of the ECG signal correlates with the CPR signal, the terminal determines, according to a degree of correlation between the noise of the ECG signal and the CPR signal, a filtering method for the first signal — S203

Whether the degree of correlation exceeds a first value — S204

Yes

No

The terminal determines to use a first filtering method for filtering the first signal — S205

The terminal determines to use a second filtering method for filtering the first signal — S206

*Fig. 7*

EP 3 741 294 B1

A terminal detects a CPR signal and a first
signal, wherein the first signal comprises
an ECG signal and noise of the ECG signal — S301

The terminal
analyzes whether the noise of the
ECG signal correlates with
the CPR signal — S302

No

If the noise of the ECG signal does not correlate with the CPR signal,
the terminal determines, according to the proportion of the noise of
the ECG signal caused by a CPR process to the first signal,
a filtering method for the first signal — S303

Yes

If the noise of the ECG signal correlates with the CPR signal,
the terminal determines, according to a degree of correlation between
the noise of the ECG signal caused by the CPR process and
the CPR signal, a filtering method for the first signal — S307

Whether the
proportion of the noise of the
ECG signal caused by the CPR process to the
first signal is greater than a
sixth threshold — S304

Whether the
degree of correlation exceeds
a first value — S308

No

The terminal determines
not to filter the first signal — S306

Yes

The terminal determines to use a third
filtering method for filtering the first signal — S305

Yes

The terminal determines to use a first
filtering method for filtering the first signal — S309

No

The terminal determines to use a second
filtering method for filtering the first signal — S310

Fig. 8

A terminal detects a CPR signal and a first signal,
wherein the first signal comprises an ECG signal
and noise of the ECG signal — S401

The terminal
ascertains whether the CPR
signal exists — S402

No

The terminal determines
not to filter the first signal — S403

Yes

The terminal
analyzes whether the noise of the ECG
signal correlates with the
CPR signal — S404

No

If the noise of the ECG signal does not correlate with the CPR signal,
the terminal determines, according to the proportion of the noise
of the ECG signal caused by a CPR process to the first signal,
a filtering method for the first signal — S405

Yes

If the noise of the ECG signal correlates with the CPR signal,
the terminal determines, according to a degree of correlation between
the noise of the ECG signal caused by the CPR process and
the CPR signal, a filtering method for the first signal — S409

Whether the
proportion of the noise of the
ECG signal caused by the CPR process to the
first signal is greater than a
sixth threshold — S406

Whether the
degree of correlation exceeds
a first value — S410

No

The terminal determines
not to filter the first signal — S408

Yes

The terminal determines to use a third
filtering method for filtering the first signal — S407

Yes

The terminal determines to use a first
filtering method for filtering the first signal — S411

No

The terminal determines to use a second
filtering method for filtering the first signal — S412

Fig. 9

25

*Fig. 10*

Terminal
**110**

Filter — 112

115

115                    115

CPR signal

ECG signal

115                    115

Processor — 111

Memory — 113

115                    115

Peripheral system

116                    117

114

Audio
controller

Display screen
controller

115                    115

Audio
circuit

Display screen
(floating touch)

118                    119

*Fig. 11*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9289134 B2